# EUROPEAN PATENT APPLICATION

(11) **EP 4 488 358 A1**
(43) Date of publication of application: **08.01.2025**
(21) Application number: 23183041.5
(22) Date of filing: 03.07.2023
(51) Int. Cl.: C12M 1/32, B01F 29/10, B01F 31/22, C12M 3/06, C12M 1/00, C12M 1/34

(54) **APPARATUS AND METHOD FOR CONTROLLABLE HANDLING OF CHEMICAL AND BIOLOGICAL MATERIAL**

(71) Applicant: Sorbus Biomedical AB, 224 79 Lund (SE)
(72) Inventor: RÖNN, Roger Emanuel, 224 79 Lund (SE)
(74) Representative: KIPA AB

(57) **Abstract**

Disclosed is a system for maintaining biological and/or chemical material under controlled environmental conditions, comprising an air-tight enclosure system having at least one gas inlet and at least one gas outlet; a gas circulation system, for delivering gas/air or mixture thereof to and from the enclosure system via the at least one gas inlet and the at least one gas outlet; a motion control system that provides the enclosure system with controlled mechanical pitch-roll orbital wave motion generated by cyclical tilting movement; and a heating system for controlling temperature within the system. Also disclosed is an apparatus for providing mixing of reactants within an enclosure, comprising a foundation for receiving one or more enclosure for holding liquid for chemical reactants and/or biological materials and a motorized component that is connected to the lower surface of the foundation, the motorized component having means to tilt the foundation with respect to a horizontal plane; and drive the foundation in a pitch-roll fashion with respect to the plane of the foundation, while preventing a yaw motion of the foundation in the plane of the foundation.

## Description

### FIELD

The disclosure relates to an apparatus for controllable handling of chemical and/or biological material, in particular in liquid form or in a liquid solution. The disclosure further relates to a method for the mixing and/or homogenization of liquids in relation to chemical and/or biological material.

### INTRODUCTION

The handling of chemical reagents in a laboratory environment typically involves use of equipment that is intended to hold the reagents and maintain or adjust conditions over time, for example by providing a supply or stream of reagents, air and/or gas mixtures to facilitate desired experimental conditions. The handling may also include control of physiochemical parameters such as light exposure, gas pressure and temperature. In some cases, a mixing action, typically mechanical, is applied to homogenize and/or mix reagents that may be provided within an open or closed environments (e.g., within closed or open reaction vessels).

The chemical reagents may alternatively or additionally include biological material, such as cell culture. Current methods for cultivating cells in a lab environment (*in vitro*) relies on standardized methods (protocols) of handling the cells and use of traditional types of equipment. These types of equipment include workbenches intended to protect cell cultures during handling from microbial contamination, and cell incubators in which cell cultures, contained in standardized plasticware, are kept and grown at appropriate temperature, humidity, and pH (which is accommodated by control of the concentration of carbon dioxide (CO₂), hence the term CO₂ incubator). Current cell culture methods furthermore frequently do not involve any active mixing of cells and their surrounding media, which inherently may result in a non-homogeneous environmental conditions within the cell culture.

### SUMMARY

The present invention seeks to overcome, eliminate and/or ameliorate one or more of the drawback and deficiencies of the prior art, for example those described in the above.

The invention relates to a system, apparatus and method for providing chemical and biological materials, with a highly controllable environment that, in addition of shielding the materials kept within from external conditions, that serves to provide control in terms such as, but not limited to, oxygen levels, rate of oxidation, carbon dioxide levels, pH in liquids, fluid dynamics, diffusion gradients, shear force, pressure conditions, and temperature conditions. The material can be kept in any convenient laboratory vessel, such as standardized laboratory plasticware.

In an aspect, there is provided a system for maintaining biological and/or chemical material under controlled environmental conditions, the system comprising (i) an air-tight enclosure system, within which the biological and/or chemical material is contained and protected from external conditions, the enclosure system enabling observation, access, handling and/or monitoring of the material without exposing the material to external air, the enclosure system having at least one gas inlet and at least one gas outlet; (ii) a gas circulation system, for delivering gas/air or any mixture thereof to and from the enclosure system via the at least one gas inlet and the at least one gas outlet; (iii) a motion control system that provides the enclosure system, and any material contained therein, with controlled mechanical pitch-roll orbital wave motion generated by cyclical tilting movement; (iv) a heating system that provides the enclosure system and/or the gas circulation system with controlled heating, thereby controlling temperature within the system.

The gas or air can comprise any single gas or mixture of gases, or a mixture of gases and air. The gas may for example be oxygen, nitrogen, argon or carbon dioxide, or any mixture thereof.

The system can furthermore comprise a gas reservoir system, for supplying the gas circulation system with one or more gases in a controlled manner. The gas reservoir system can comprise one or more gas containment vessel that can be provided gas for example from one or more gas tank or other gas supply that is connected to the system by one or more gas supply lines.

The gas supply may for example comprise at least one gas containment vessel for providing a gas and/or air mixture containing humidified air. The gas reservoir system may for this purpose comprise one or more humidifier, such as a heated humidifier. Such a humidifier can comprise a volume of water that, when heated, provides the gas reservoir system with humidified air.

The gas and/or air mixture can be supplied at any suitable pressure, such as pressure in the range of 0-300 kPa. By way of example the gas and/or air mixture can comprise carbon dioxide gas from a supply at 0-300 kPa and/or nitrogen from a supply that is at 0-300 kPa. The gas circulation can provide for sterile transportation of the prepared gas mixture between the enclosure system and the reservoir system. Gas flow rates can generally be in the range of 0-1000 mL/min, such as in the range of 0-10mL/min, 0-100 mL/min and 0-500 mL/min.

The gas reservoir system may further comprise one or more of the following: one or more sensors for determining concentration of one or more gases; ports and valves for injection of pressurized gas; inlet and outlet ports for delivering gas mixtures to and from the gas reservoir system; and one or more fans for providing mixing of air/gas in the system.

The enclosure system can comprise any convenient enclosure for holding chemical and/or biological reactants. The enclosure can be adapted to receive and hold reactants, or the enclosure system can be adapted to receive standardized laboratory incubation vessels that are designed to hold reactants. For example, the enclosure system can comprise (i) an enclosure bottom that is adapted to receive standardized laboratory incubation vessels; and (ii) an enclosure lid that is adapted to encircle and meet an upper surface of the standardized laboratory incubation vessel so as to provide an air-tight seal between the enclosure lid and the incubation vessel.

The enclosure bottom and/or the enclosure lid can comprise or consist of material that is transparent to visible light, so that chemical and/or biological material within the enclosure can be observed during operation of the system.

The enclosure lid can comprise at least one air-tight sensor port for enabling the introduction of one or more sensors to measure one or more parameter within the enclosure. The parameters that are measured can for example be selected from gas concentration, temperature, pH, pressure and osmolarity.

Gas pressure within the enclosure may be maintained at any desirable pressure, which is typically in the range of 100-300kPa. For example, the gas pressure can be maintained at or near atmospheric pressure (101.32 kPa +/- 30 kPa).

The enclosure lid can further comprise one or more self-sealing ports, for enabling the insertion or extraction of material into or from the incubation vessel without exposing the interior of the enclosure to external air. Such self-sealing ports can comprise or consist of any suitable self-sealing material. Self-sealing materials have the ability to automatically repair damages to themselves without an external diagnosis or external intervention. Self-sealing materials include self-sealing polymers and elastomers such as natural or synthetic rubber materials.

The circulation system can comprise one or more pumps, for moving air or gas mixture from the gas reservoir system into the enclosure system. The circulation system can furthermore comprise one or more filters, for filtering air or gas in the system.

The heating system can be provided through use of one or more heating plates that provide heating to the enclosure system. Thus, the heating system can comprise one or more heating plate that is adapted to meet the enclosure bottom, thereby providing heating to the bottom of the enclosure and thereby to any chemical and/or biological material within the enclosure. For example, when the enclosure is adapted to receive standardized laboratory vessels, the heating system provides the laboratory incubation vessels within the enclosure with controlled heating.

Alternatively, or additionally, the heating system can provide heating to the enclosure lid. For example, the heating system can further comprise heating means that is integrated into the enclosure lid, for providing the enclosure with controlled heating and preventing condensation on the inner surface of the enclosure lid. The heating system can additionally or alternatively provide heating to one or more additional components of the system. For example, the heating system can further comprise heating means that is integrated into, or connected to, the gas containment vessel of the reservoir system so as to provide heating of gas/air in the gas containment vessel. The heating system can further comprise heating means that is integrated into circulation filters and/or fittings for circulation filters in the system. The heating system can further comprise heating means that is integrated into/onto the circulation system, to thereby provide heating to gas/air within the circulation system. The heating system can generally control heating within the system, e.g. the enclosure system, the reservoir system and/or the circulation system, to any desired temperature that is in the range of about 5°C - 75°C, such as about 10°C - 65°C, 15°C - 50°C, 15°C - 40°C, 20°C - 37°C, 25°C - 37°C or 30°C - 37°C. In some cases, the desired temperature is ambient temperature. Cooling to temperatures below typical room temperature can be carried out for example by placing the system within a cooled environment, e.g. a cooled room. Thereby, the system can be operated at the desired temperature using the internal heating mechanism in the system.

Another aspect relates to an apparatus for providing mixing of liquid chemical reactants or biological culture within an enclosure through a pitch-roll orbital wave motion generated by controlled cyclical tilting movement. The apparatus comprises (i) a base component, comprising a central shaft oriented in a z-direction perpendicular to a flat resting surface and a drive train for providing rotational movement around the central shaft; (ii) a rotational component, the rotational component being operatively connected to the drive train so that the rotational component can rotate around the z-axis, the rotational component having an angled upper portion so as to form an angled wedge when viewed from the side, the upper portion following the rotational movement around the central shaft as the rotational component rotates; (iii) a foundation, the foundation having a lower surface that meets the angled upper portion of the cylindrical rotational component and an upper surface, for receiving one or more enclosure comprising chemical reactants and/or biological liquids; (iv) an arrangement of bearings connecting the foundation to the rotational component so that the bearings form an interface between the rotational component and the lower surface of the foundation; and (v) a linker component that is operatively connected to the platform, the linker component allowing pitch-roll motion of the foundation while preventing yaw motion of the foundation. When in operation, the foundation follows the repositioning of the rotational component without itself rotating around the central shaft. This results in a pitch-roll orbital wave motion of the foundation and any enclosure placed on the foundation.

The rotational component thus rotates around the central shaft of the base component when the apparatus is operating, driven by the drive train. The rotational component can be angled at its upper end, i.e. the upper end portion of the rotational component is angled when viewed from the side.

The upper surface of the foundation can be largely flat, so as to be able to accommodate conventional laboratory plasticware. The foundation can have grooves or protrusions that serve the role of positioning such plasticware or other appropriate enclosures containing liquid chemical reactants or biological culture on the foundation.

The bearings can be arranged on the cylindrical rotational component at or near the upper end thereof, the bearings being aligned with the angled wedge of the upper end. Alternatively, the bearings can be arranged on, or attached to, the lower surface of the foundation. In either case, the bearings provide for the pitch-roll motion of the foundation as the rotational component rotates, without the foundation itself rotating.

The linker component can preferably contain, or consist of, elastic material that provides for the movement of the connected foundation in a pitch-roll fashion. The linker component can comprise an elastic disk with an aperture for connecting to the central shaft, thereby forming a connection between the central shaft and the foundation, the linker providing flexibility to movement of the foundation in terms of pitch and roll, while preventing yaw movement. Thus, the linker component can be connected to the central shaft so that the foundation is secured to the non-rotating central shaft. Through the flexibility of the linker component and the connection of the linker component to the rotational component via the bearings, the foundation is moved in a pitch-roll fashion as the rotational component rotates.

In a further aspect, the invention relates to a method of controlled mixing of liquid chemical reactants or biological culture. The method comprises the following steps that are preferably performed in order:
- providing an air-tight enclosure, wherein the enclosure is adapted to be disposed on a solid foundation that is connected to a mechanical assembly for controlled movement of the foundation, and wherein the air-tight enclosure has one or more gas connections for receiving one or more gas mixture without exposing the interior of the enclosure to external air;
- disposing liquid chemical and/or biological material into the air-tight enclosure;
- controlling the composition of gas within the enclosure by supplying gas through the one or more gas connection; and
- subjecting the enclosure to a pitch-roll movement without rotating the enclosure, thereby facilitating mixing of chemical and/or biological components within the enclosure while minimizing force needed to facilitate mixing.

The air-tight enclosure can be adapted for receiving one or more receptacle for containing the one or more cell culture, and wherein the one or more cell culture is disposed into such one or more receptacle within the enclosure. The one or more receptacle can be provided by standardized laboratory vessels such as laboratory plasticware, including for example disposable plasticware.

The method can further comprise include controlling the temperature of the air-tight enclosure and any liquid chemical and/or biological material therein by means of a heating assembly. The heating assembly can be connected to the solid support. For example, the heating assembly can consist of, or contain, one or more heating plates for receiving the air-tight enclosure.

The method can be preferably carried out using the system and apparatus described further herein.

Exemplary embodiments in accordance with the invention include:
1. A system for maintaining biological and/or chemical material under controlled environmental conditions, the system comprising:
   i. an air-tight enclosure system, within which the biological and/or chemical material is contained and protected from external conditions, the enclosure system enabling observation, access, handling and/or monitoring of the material without exposing the material to external air, the enclosure system having at least one gas inlet and at least one gas outlet;
   ii. a gas circulation system, for delivering gas/air or mixture thereof to and from the enclosure system via the at least one gas inlet and the at least one gas outlet;
   iii. a motion control system that provides the enclosure system, and any material contained therein, with controlled mechanical pitch-roll orbital wave motion generated by cyclical tilting movement;
   iv. a heating system that provides the enclosure system and/or the gas circulation system with controlled heating, thereby controlling temperature within the system.
2. The system of embodiment 1, further comprising a gas reservoir system, for supplying the gas circulation system with one or more gases in a controlled manner.
3. The system of the preceding embodiment, wherein the gas reservoir system comprises at least one air containment vessel for providing a gas mixture of humidified air.
4. The system of the preceding embodiment, wherein the gas reservoir system further contains one or more of: sensors for determining concentration of one or more gases; ports and valves for injection of pressurized gas; inlet and outlet ports for delivering gas mixtures to and from the gas reservoir system; and one or more heated humidifier.
5. The system of any one of the preceding embodiments 1-4, wherein the enclosure system comprises:
   i. an enclosure bottom that is adapted to receive standardized laboratory incubation vessels;
   ii. an enclosure lid that is adapted to encircle and meet an upper surface of the standardized laboratory incubation vessel so as to provide an air-tight seal between the enclosure lid and the incubation vessel.
6. The system of the preceding embodiment, wherein the enclosure bottom and/or the enclosure lid are transparent to visible light, so that material within the enclosure can be observed during operation of the system.
7. The system of any one of the preceding two embodiments 5-6, wherein the enclosure lid comprises at least one air-tight sensor port for enabling the introduction of one or more sensors to measure one or more parameter within the enclosure selected from gas concentration, temperature, pH, pressure and osmolarity.
8. The system of any of the preceding three embodiments 5-7, wherein the enclosure lid further comprises one or more self-sealing ports, for enabling the insertion or extraction of material into or from the incubation vessel without exposing the interior of the enclosure to external air.
9. The system of any one of the preceding embodiments 1-8, wherein the circulation system comprises one or more pumps, for moving air or gas mixture from the gas reservoir system to the enclosure system.
10. The system of any one of the previous embodiments 1-9, wherein the circulation system comprises one or more filters, for filtering air or gas mixture in the system.
11. The system of any one of the previous embodiments 5-10, wherein the heating system comprises a heating plate that is adapted to meet the enclosure bottom, thereby providing laboratory incubation vessels within the enclosure with controlled heating.
12. The system of any one of the previous embodiments 5-11, wherein the heating system further comprises heating means that is integrated into the enclosure lid, for providing the enclosure with controlled heating and preventing condensation on the inner surface of the enclosure lid.
13. The system of any one of the previous embodiments 5-12, wherein the heating system further comprises heating means that is integrated into the air containment vessel of the reservoir system.
14. The system of any one of the previous embodiments 5-13, wherein the heating system further comprises heating means that is integrated into circulation filters and/or fittings for the circulation filters.
15. The system of any one of the previous embodiments 5-14, wherein the heating system further comprises heating means that is integrated into tubing or air/gas passageways in the circulation system.
16. An apparatus for providing mixing of liquid used with chemical reactants or biological culture within an enclosure through a pitch-roll orbital wave motion generated by controlled cyclical tilting movement, the apparatus comprising:
   i. at least one foundation having a lower surface and an upper surface for receiving one or more enclosure for holding liquid for chemical reactants and/or biological materials;
   ii. a motorized component that is connected to the lower surface of the foundation, the motorized component having means to:
      i. tilt the foundation with respect to a horizontal plane; and
      ii. drive the foundation in a pitch-roll fashion with respect to the plane of the foundation, while preventing a yaw motion of the foundation in the plane of the foundation,
   whereby the motorized component when in use drives the foundation along a pitch-roll orbital path so that any enclosure positioned on the upper surface of the foundation follows the same motion.
17. An apparatus for providing mixing of liquid chemical reactants or biological culture within an enclosure through a pitch-roll orbital wave motion generated by controlled cyclical tilting movement, the apparatus comprising:
   i. a base component, comprising a central shaft oriented in a z-direction perpendicular to a flat resting surface and a drive train for providing rotational movement around the central shaft;
   ii. a rotational component, the rotational component being operatively connected to the drive train so that the rotational component can rotate around the z-axis, the rotational component having an angled upper portion so as to form an angled wedge when viewed from the side, the upper portion following the rotational movement around the central shaft as the rotational component rotates;
   iii. a foundation, the foundation having a lower surface that meets the angled upper portion of the cylindrical rotational component and an upper surface, for receiving one or more enclosure comprising chemical reactants and/or biological liquids;
   iv. an arrangement of bearings connecting the foundation to the rotational component so that the bearings form an interface between the rotational component and the lower surface of the foundation; and
   v. a linker component that is operatively connected to the platform, the linker component allowing pitch-roll motion of the foundation while preventing yaw motion of the foundation;
   wherein when the apparatus is in operation the foundation follows the repositioning of the rotational component without itself rotating around the central shaft, thereby resulting in a pitch roll orbital wave motion of the foundation and any enclosure placed on the foundation.
18. The apparatus of the previous embodiment 17, wherein the bearings are arranged on the cylindrical rotational component at or near the upper end thereof, the bearings being aligned with the angled wedge of the upper end.
19. The apparatus of embodiment 17, wherein the bearings are arranged on or attached to the lower surface of the foundation.
20. The apparatus of any one of the previous embodiments 17-19, wherein the linker component comprises an elastic disk with an aperture for connecting to the central shaft, thereby forming a connection between the central shaft and the foundation, the linker providing flexibility to movement of the foundation in terms of pitch and roll, while preventing yaw movement.
21. The apparatus of any one of the previous embodiments 17-20, wherein the bearings are arranged on the rotational component, and wherein the linker component further comprises a non-elastic disk-shaped portion having a central opening within which the elastic disk is positioned.
22. A method of controlled mixing of liquid chemical reactants or biological culture, the method comprising:
   - providing an air-tight enclosure, wherein the enclosure is adapted to be disposed on a solid foundation that is connected to a mechanical assembly for controlled movement of the foundation, and wherein the air-tight enclosure has one or more gas connections for receiving one or more gas mixture without exposing the interior of the enclosure to external air;
   - disposing liquid chemical and/or biological material into the air-tight enclosure;
   - controlling the composition of gas within the enclosure by supplying gas through the one or more gas connection;
   - subjecting the enclosure to a pitch-roll movement without rotating the enclosure, thereby facilitating mixing of chemical and/or biological components within the enclosure while minimizing physical force needed to facilitate mixing.
23. The method of the previous embodiment 22, wherein the air-tight enclosure is adapted for receiving one or more receptacle for containing the one or more cell culture, and wherein the one or more cell culture is disposed into such one or more receptacle within the enclosure.
24. The method of any one of the previous two embodiments 22-23, further comprising controlling the temperature of the air-tight enclosure and any cell culture therein by means of a heating assembly that is connected to the foundation.
25. The method of any one of the previous three embodiments 22-24, wherein the method is carried out using a system according to any one of embodiments 1-15.

### BRIEF DESCRIPTION OF THE DRAWINGS

**FIG. 1** shows an illustration of pitch, roll and yaw motion.
**FIG. 2** shows an exemplary enclosure.
**FIG. 3** shows an exemplary heating plate.
**FIG. 4** shows in (A) -(E) an illustration of various components of a motion control apparatus.
**FIG. 5** shows a system wherein the tilt of the motion control apparatus can be varied.
**FIG. 6** shows a air/gas delivery system that can be used with the system described herein.
**FIG. 7** shows an air/gas reservoir system that includes a body of water for controlling humidity.
**FIG. 8** shows a top view of a system that can accommodate up to four enclosures.

### DESCRIPTION

The present disclosure relates to an apparatus designed to provide environmental control of liquid chemical and/or biological material, by providing an environment in which a dynamic and highly controlled environment serves to provide liquid chemical and/or biological material with an environment that maximizes the yield and/or efficiency. This is achieved by a combining factors that provide enhanced mixing of liquid reactants while minimizing shear forces, and at the same time providing a controlled environment through control of gas pressure/composition, heat etc. For example, biological cells can by use of the apparatus be provided with protection from stress and functional decline that otherwise occur with traditional equipment for cell cultivation.

Successful cultivation of cells is often not guaranteed, with many types of cells being difficult to successfully cultivate while maintaining their functional value, often resulting in failed experiments or undesired variability. One major cause for this difficulty is that cells easily get stressed and react negatively when they are exposed to conditions that differ from their natural environment, for example inside the human body. Such stress may result in cellular functional decline, which in turn limit the usefulness and application potential of cultivated cells in various life science applications, with examples including poor reliability of cells for analytical purposes, reduced yields when cells are used for production of drugs or biological substrates, or an inability to expand functional cells intended for therapeutic applications in healthcare.

For example, it is well documented that an overexposure of oxygen to cells may cause increased amounts of oxidative damage to cellular components such as DNA, proteins, and lipids. When working with traditional methods and equipment for cell cultivation, oxygen overexposure occurs as a result of to the cells being handled and kept in gas conditions that are equivalent or similar to that of the atmosphere. Depending on the cell type (and their inherent resilience to such stress) the negative consequences of such oxidative stress may range from mild to critical, with the latter resulting in the death or functional decline of the cell.

While oxygen overexposure has a damaging effect on cells, oxygen is still a central component needed for cells to carry out metabolism, with cells absorbing dissolved oxygen present in their immediate environment, and situations where the minimal demand for oxygen cannot be provided is known to compromise cellular survival.

The apparatus can provide superior reaction conditions by creating a controlled chemical environment that supplies reactants in desired quantity over time and at the same time providing a dynamic fluid environment that ensures adequate mixing, and in particular avoids formation of diffusion gradients within the reaction solution.

For example, the apparatus can protect cultivated cells from stress and functional decline by recreating two main factors provided to the cells in their natural body environment. These two factors are: 1) providing an oxygen concentration that does not exceed its natural environment, e.g. that of the human or animal body, and 2) providing a dynamic movement of fluid that prevent diffusion gradients of nutrients or oxygen from establishing. Together, these two factors allow the assertion of real control over cellular oxygen and/or nutrient exposure, enabling successful cultivation at body-like (physiological) conditions that reduce the functional decline that otherwise occur in cells when using traditional cell cultivation equipment.

Oxygen (O₂) is an important factor for life and cellular function but is also a powerful chemical reactant. While available at a gaseous concentration of approximately 21% in the normal atmosphere, it is also found as a dissolved factor in liquids where an equilibrium between gaseous and dissolved oxygen may form. If allowed to fully equilibrate, an aqueous solution that is in contact to the atmosphere is said to contain an oxygen concentration equivalent to 21% O₂. The amount of dissolved oxygen that exist in the body (the cells natural niche) is significantly lower, often measured at between <1% to 3% O₂ (depending on the exact body location) and is being continuously provided to the tissues of the body from the lungs via the circulatory system.

An important (and often overlooked) flaw with traditional methods of cell cultivation is how the cultivated cells are kept during culture. During cell cultivation, plasticware containing cells are kept standing still (statically) on shelves within the typical CO₂ incubator. While the cells natural environment in the body is a highly dynamic system (with the blood stream providing circulation and controlled turnover of the extracellular fluid), what occurs when cells are kept in traditional cell incubators is that the fluid is static. As a consequence, the primary way that dissolved factors in a static fluid can move will be based on passive diffusion, which puts a limitation on how fast oxygen may move to the immediate vicinity of the cell.

If the consumption rate of oxygen from the cells would increase (which does occur when the cells increase their numbers through cell division) the amount of oxygen that is available to each cell in such a static system will change. Due to the inherent formation of diffusion gradients in static systems, as well as due to the inherently changing rate of oxygen consumption from growing numbers of cells, the result is that traditional systems of cell cultivation leaves the user without any sense of control over the real degree of cellular oxygen exposure, which result in suboptimal oxygen exposure causing stress and functional decline in the cultivated cells.

The invention differs from earlier types of equipment due to combined application of 1) providing the ability to control the oxygen concentration from atmospheric levels, for example by bringing oxygen levels towards that of the body, and 2) providing a dynamic movement of fluid that prevent diffusion gradients from occurring. Together, these two factors enable successful and controlled cell cultivation of cells at concentrations of dissolved oxygen equal to what is found inside the body, without cellular functionality or survival becoming compromised due to overexposure or underexposure to oxygen.

To carry out procedures to incubate liquid chemical and/or biological material with the invention, plasticware intended for containing the reagents (chemical reagents and/or biological cell cultures, often standardized multi-well plates or Petri dishes) are used together with a specialized bottom (enclosure bottom) and top (enclosure lid) that together form a containment vessel or enclosure. An important feature of this containment vessel includes the ability to use microscopy or other means to observe material kept within the vessel, without compromising the internal environment, due to the design of the bottom part. For example, cells can be observed through the containment vessel during their incubation and growth using conventional microscopy means to observe material at any desirable magnification, such as 4X, 10X, 20X, or greater. Another important feature is the ability to interact with the material within the enclosure without compromising the internal environment of the enclosure, by use of perforating novel self-sealing plugs installed in the lid part.

The rate of the pitch-roll orbital wave motion can be controlled to provide the desired motion of and thereby mixing of material within the enclosure system/containment vessel. For example, the pitch-roll orbital wave motion can be provided at 0-100 rpm, including any number within the range such as 0-80rpm, 0-60 rpm, 0-50 rpm or 0-40 rpm or 0-30 rpm or 0-20 rpm. The motion can be controlled as desired during the incubation of material within the enclosure system/containment vessel.

Once the enclosure is assembled it can be placed onto a heating block that provides direct and controllable temperature to the liquid chemical and/or biological material (e.g., cells) kept within the enclosure.

To provide the environment within in the enclosure with a controlled gas mixture, a 'reservoir unit' in which sensors, gas injectors, and fans together prepare a volume of air with the desired proportion of gases, is available. Prepared gas mixture is circulated to and from the enclosure and the reservoir unit by the use of pumps, thereby facilitating the desired gas mixture in the enclosure during the incubation.

To provide the contents of the enclosure with a dynamic movement of fluid to prevent diffusion gradient from occurring the enclosure (together with the specialized heating block if present) is placed on a mechanical platform that provides a pitch roll orbital wave motion. This movement facilitates controllable stirring of the liquid within the enclosure which assures uniformity across the liquid volume in terms of dissolved oxygen (or other gas) content.

The invention provides several distinct advantages as compared to traditional equipment. Due to the design and capabilities of the enclosure or containment vessel, it is possible to carry out procedures, such as cell cultivation and handling of liquid chemical and/or biological material without the need to expose the material to unwanted environmental changes and fluctuations which otherwise occur when material is exposed to external conditions or taken in and out of the incubator for handling.

Further, by virtue of having a closed loop structure of the gas circulation system, the invention does not suffer from losses of gas mixture that otherwise occur every time an incubator needs to be opened to access material within the incubator.

The invention also provides distinct advantages when compared to other forms of equipment such as bioreactors. While bioreactors feature a high degree of environmental control (which may be used to recreate aspect of the natural cellular environment inside the body) these devices are not applicable for cultivating cells in traditionally used laboratory plasticware (such as multi-well plates and Petri dishes) frequently used with in standardized cell cultivation. Furthermore, bioreactors generally prevent the user from accessing and handling the cultivated cells in ways comparable to traditional cell cultivation in the lab, and are limited in their ability to support direct cellular observation through microscopy.

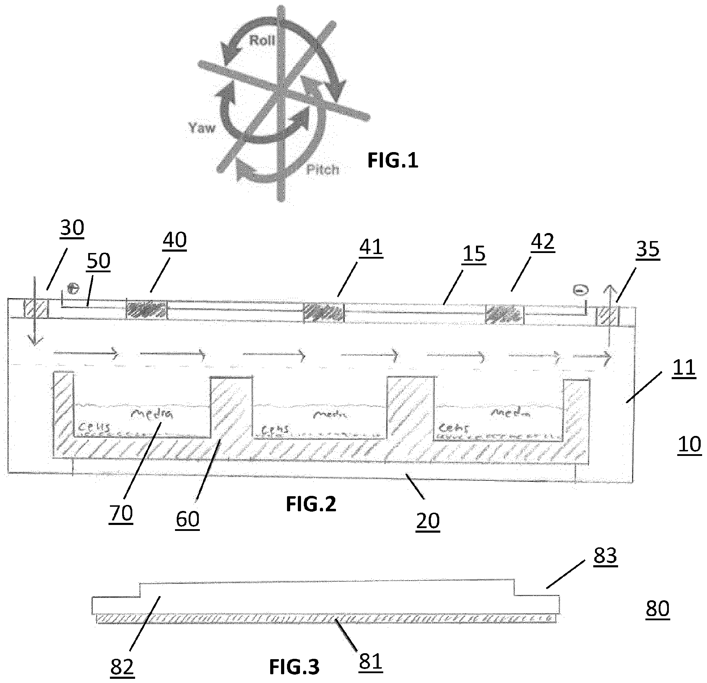

Turning now to **FIG. 1****,** the movements of pitch (tilting forward and backward), roll (tilting side to side), and yaw (tilting left and right) are illustrated. The system and apparatus described herein provides for a motion that includes a pitch and roll motion, i.e. tilting forward and backward, as well as tilting to the sides, while at the same time preventing yaw motion, i.e. motion in the plane of the enclosure system or a platform/foundation on which the enclosure system is resting. This way, an orbital wave motion is generated that results in favorable yet gentle mixing of liquid reagents within an enclosure as described further herein.

In **FIG. 2****,** a side view of an enclosure system **10** is illustrated. The enclosure system has a main body **11,** a lid portion **15** and a bottom portion **20.** An import port **30** and an export port **35** are provided in the lid portion, for respectively receiving and releasing air/gas into and from the enclosure. The lid portion and main body together serve to isolate the enclosed part(s) from the external environment, by forming a gas-tight seal/boundary to the upper part of the enclosed plasticware This way, the air/gas import and export ports can be used to control the composition of the air/gas within the enclosure, as well as the humidity.

The lid portion has three access plugs **40, 41, 42** located above the wells/vessels provided by the cultivation plasticware, for injecting or removing samples from the enclosure. The access plugs can also be used for inserting sensors into the system. The access plugs are preferably self-sealing, allowing for sample delivery or removal by way of a needle or the like without exposing the interior of the enclosure to external conditions/air. This way the enclosure can remain air-tight at all times during its operation. The lid portion has a heating wire **50** extending along the lid. The heating wire is shown to be embedded within the lid material, although it is possible to position the heating wire at or near the inner our outer surface of the lid.

Cultivation plasticware **60** is contained within the main body of the enclosure, so that the plasticware rests on at least a part of the bottom portion **20.** Liquid reagents, such as growth media for suspended or adherent cells are contained within wells **70** of the plasticware **60.** During operation of the mixing apparatus the enclosure system and the plasticware placed within the enclosure, undergo a pitch-roll orbital movement that provides for the mixing of the reagents within the wells in the enclosure.

The lid portion and/or the bottom portion can be comprised of transparent material, i.e. material that is transparent to visible light. This way, the interior of the enclosure can be viewed through the lid portion and/or the bottom portion. Alternatively, the bottom portion can be at least partially open so that that cultivation plasticware placed within the enclosure can be viewed directly from below.

The enclosure system can be a one-piece design, i.e. the enclosure system itself can contain individual wells for placing chemicals/cells for reacting and/or culturing. When so designed, the enclosure is thus self-contained in that it contains all the features necessary to hold liquids for mixing within a closed environment that is maintained by controlled air/gas delivery through the air/gas import and export ports and the optional heating wires in the lid portion of the enclosure. When so designed, the enclosure will have a solid bottom portion that can be preferably made from (at least partially) transparent material to allow the contents of the enclosure to be monitored and/or viewed from below.

Heating to the enclosure can additionally or alternatively be provided by means of a heating plate on which the enclosure rests. One such heating plate is shown in **FIG. 3****.** The heating plate **80** has a main body in the form of a metal plate **81** that can be made from any suitable metal or metal alloy that conducts heat well, such as but not limited to aluminum or steel alloys. The metal plate can have an upper surface that is designed to conform to the lower bottom of the enclosure, shown here by grooves **83** on the outer edges of the heating plate. Additional or other grooves or protrusions can be provided in the metal plate to accommodate enclosures with other shapes.

The lower surface of the heating plate **80** has an electric heating pad **80.** This heating pad provides heat to the plate, driven by electricity. Any suitable heating pad or heating element can be used for this purpose. The heating pad can for example be an adhesive silicone heater mat, such as those supplied by http://rs-online.com.

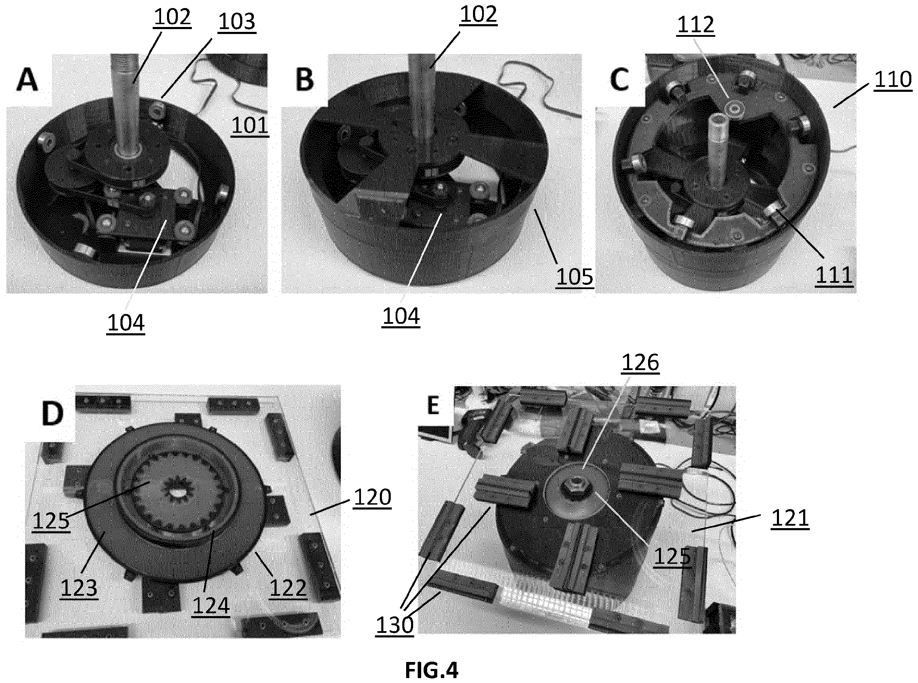

In **FIG. 4** an exemplary motion control system that is designed to generate an orbital pitch-roll movement placed thereon. In (A), a base component **101** is shown in the form of a generally cylindrical housing having a central shaft **102** attached thereto. The base component furthermore has a drive train **104** that is designed to generate circular movement around the central shaft **102.** In (B), a first rotational component **105** is shown, the rotational component having a generally cylindrical shape with the upper end having a larger diameter than the lower end. The central shaft **102** extends through the rotational component. The rotational component attaches to, and rests on, the base component **101.** Attachment of the rotational component to the drive train **104** results in rotational movement of the rotational component around the central shaft **102** during operation of the drive train **104.** Rotation of the rotational component with respect to the base component is facilitated by bearings **103** on the based component that meet a lower end surface of the rotational component, allowing the rotational component to rotate with respect to the stationary base component **101.**

In (C), a second rotation component **110** is shown. This second rotational component is generally cylindrically shaped and sits on top of the first rotational component **105,** with the central shaft **102** extending through the second rotational component **110.** The upper end of the rotational component **110** is slanted so that when viewed from the side, the upper end of the rotational component **110** has a wedge-like shape. Bearings **111, 112** are provided near the upper end of the rotational component, to facilitate the rotational movement of the rotational component with respect to foundation or platform placed thereon.

In (D), the lower side of a foundation **120** is shown. This foundation has a generally flat upper surface, on which one or more enclosure can be accommodated. On the lower surface of the foundation, a linker component **122** is shown. The linker component has a disk-like portion **123** with a central opening. In this central opening an elastic disk **125** having a central aperture is attached. The central aperature is designed to allow the central shaft of the base component to extend therethrough.

The disk-like portion of the linker component serves the purpose of meeting the bearings **111, 112** of the rotational component so that the rotational component can freely rotate with respect to the non-rotating foundation **120.** A circular protruding portion **124** on the linker component is provided so that bearings **112** can meet an inner surface thereof, facing the central aperture, while bearings **111** meet the generally disk shaped outer portion **123** of the linker component.

The foundation **120** is secured to the central shaft by a nut **126,** as indicated in (E), where the assembled apparatus is shown from above. The nut can be replaced by other lock designs known in the art. The upper surface **121** of the foundation is generally flat, with protrusions **130** in the shape of stoppers being provided to accommodate enclosures thereon. In the apparatus shown in (E), four enclosures can be accommodated on the upper surface of the foundation. The elastic disk portion **125** of the linker component **122** can be seen through the central opening in the disk-like portion **123.**

It should be appreciated that the first and second rotational components **105, 110** can be provided as a single rotational component, through which the central shaft **102** extends. This single rotational component can preferably be generally cylindrically shaped with a wedgedlike upper end, so that a foundation or platform attached thereto is tilted with respect to the surface on which the apparatus rests.

The foundation **120** is secured to the stationary central shaft **102.** This has the consequence that as the rotational components rotate, driven by the drive train, the foundation will be forced to adapt to the rotational movement of the wedge-like upper portion of the rotational component **110.** The elastic disk portion **125** of the linker component **122** allows such a motion so that the foundation can follow the rotation of the rotational component, facilitated by the bearings **111,112** on the rotational component and bearings **103** on the base component. The consequence is that the foundation, and any material placed thereon, will follow a movement that results in motion back and to the sides, i.e. a pitch-roll orbital motion. At the same time, there is no yaw motion of the foundation, i.e. side-to-side motion in the plane of the foundation.

It will be appreciated that bearings **111, 112** shown here to be provided on the rotational component, can alternatively be provided on the foundation **120.** When so designed, the rotational component will have a receiving surface so that the rotational component can move freely against bearings on the (non-rotating) foundation.

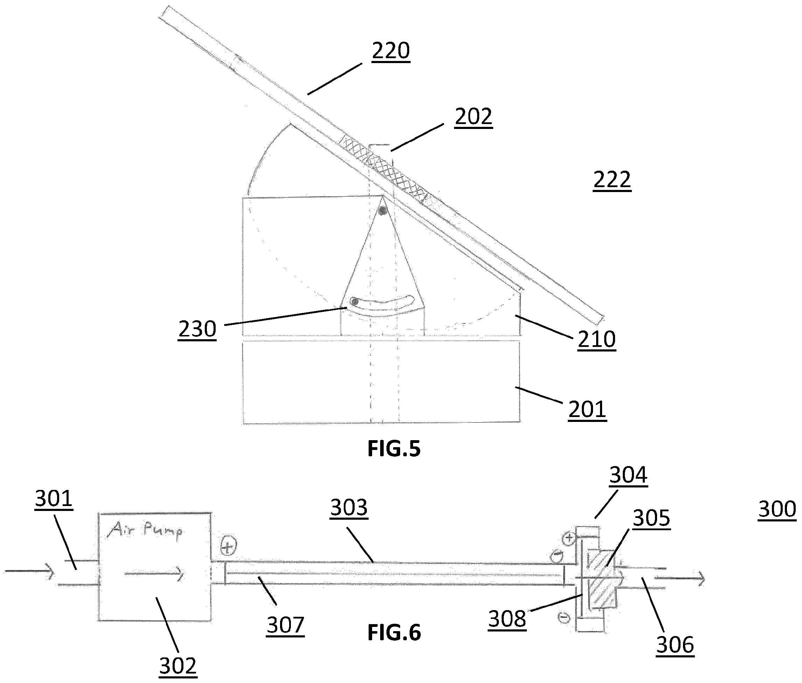

In **FIG. 5****,** an alternative motion control apparatus **222** is shown. The general mechanical construct is similar to that of the apparatus shown in FIG.4. There is thus a base component **201,** a rotational component **210** and a platform-shaped foundation **220** resting on the rotation component **210.** A central shaft **202** extends through the apparatus, being fixed to the base component and the foundation. There is further a tilting mechanism **230** shown, for varying the tilt of the foundation **220** with respect to ground. The tilting mechanism can adjust the tilt of the foundation to any desired angle with respect ground, i.e. an angle in the range of 0° to 60°, such as 0° to 50°, 0° to 45°, 0° to 40° or 0° to 30°. Thereby, an added variable is provided for ensuring proper mixing of liquid reagents or reagents provided within a cultivation plasticware, depending on the nature of the components, their viscosity, concentration or shape/volume of the container within which the components are placed (i.e., the enclosure, or plasticware placed within the enclosure).

It becomes apparent that for small angles, i.e. an angle at or near 0°, the foundation will only move slightly or not at all as the rotational component moves. However, with increased angle, the degree of movement will increase thereby providing increased movement and hence mixing of reagents/liquids within the enclosure. Thereby, through the combination of the tilting angle and the rotational speed, the degree of mixing can be varied at will.

In **FIG. 6****,** an exemplary air/gas circulation system is shown. The gas circulation system **300** can circulate any air gas mixture through the overall system, driven by one or more air pump **302** having an air/gas entry point **301.** The air/gas flows through tube **303,** which is shown to have an (optional) air/gas filter holder **304,** within which one or more filters **305** are placed. There is an exit point **306,** from which air/gas exits the circulation system and into one or more enclosure (not shown). An electrical heating wire **307** extends along the tube **303,** with a second wire **308** providing heating to the air filter holder **304,** thereby allowing heating of air/gas within the system, to prevent condensation of water vapor that may clog the filter and prevent circulation. This ensures that temperature can be controlled throughout the overall system, i.e. the enclosure and air/gas delivery system that delivers gas into the enclosure.

Alternatively or additionally the gas circulation system may incorporate hollowed out sections of material (metal, plastics, etc.) through which the air/gas mixture may travel. These sections of hollowed out material may also be heated by use of heating pads or similar.

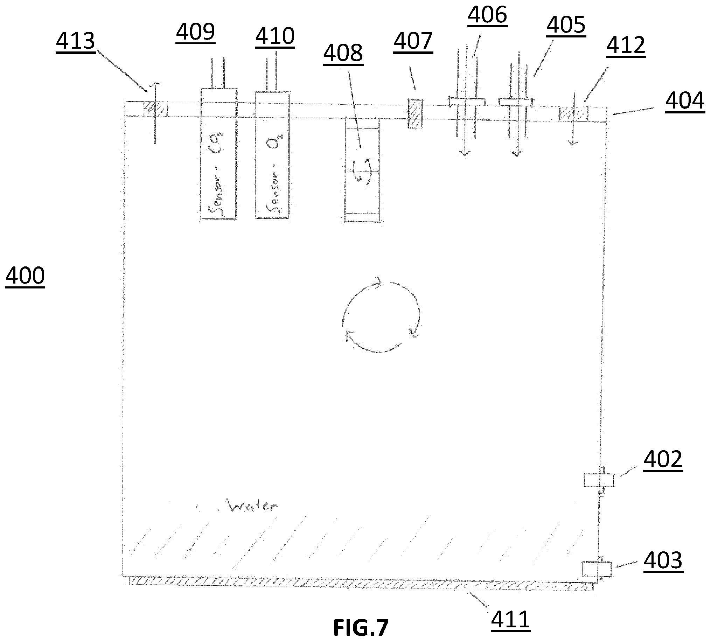

An exemplary gas reservoir system is shown in **FIG. 7****.** The system consists of an enclosure **401** having a lid **404.** The enclosure has a water import port **402** and a water drainage **403,** for delivering water into and draining water from the system, respectively. During operation, there will be a partial supply/amount of water **420** within the enclosure, i.e. the enclosure will be partially filled with water. At the bottom of the enclosure there is a heating pad **411,** which serves the purpose of heating the water in the enclosure, thereby increasing the humidity of gas within the enclosure.

The enclosure has ports for supplying and releasing air and/or gas, shown here to extend through the lid **404.** Thus, there is an air injection port **412** that can be used to deliver air or air/gas mixtures into the enclosure and an air export port **413** for releasing air or air/gas from the enclosure. Shown also are a nitrogen injection port (for the purpose of lowering the proportion of oxygen in the gas mixture) **405** and a carbon dioxide injection port **406,** for supplying the enclosure with supplementary carbon dioxide. A pressure escape port **407** allows depressurization of the enclosure as needed.. A fan **408** circulates air/gas within the enclosure, with sensors **410, 411** determining the concentration of gases such as oxygen and carbon dioxide within the enclosure.

The gas reservoir system, when in operation, can thus be used to supply the overally system with air/gas of any desired composition having any desired humidity, by controlling the degree of heating of water within the enclosure and the partial pressures of oxygen and carbon dioxide. Thereby, air/gas supply can be regulated within the system.

There can also be a temperature sensor in the enclosure, to monitor the temperature of water within the enclosure.

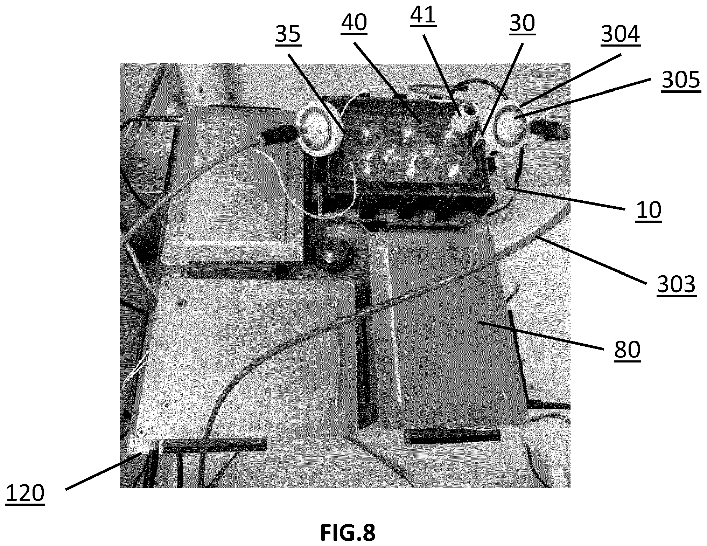

In **FIG. 8****,** an exemplary system is shown, referring also to **FIGs. 1-3****.** In the view show, one enclosure **10** has been placed on foundation **120** on top of a heating plate **80** that is provided on the upper surface of the foundation. The enclosure has air/gas import and export ports **30, 35** through which air/gas is supplied from a gas reservoir system (not shown). Two variations of self-sealing plugs **40, 41** are shown, for delivering material (e.g., reagents, media, cells) or sensors into the enclosure or plasticware placed within the enclosure. Connected to the enclosure is part of the air/gas circulation system, including air/gas filter holder **304,** filter **305,** and tubing **303.** During operation of the system, up to four enclosures can be provided on the foundation, each enclosure operating at the same or different experimental conditions. The enclosures are all driven along the same pitch-roll path, driven by the motion control system, only the foundation part thereof (upper platform) being shown in this view.

The air/gas circulation system can at least partially contain hollowed out material (for example plastic or metal) that functions as passageways for gas/air within the system. Thus, tubing for delivering gas can at least partially be replaced by hollowed out material.

As used herein, including in the claims, singular forms of terms are to be construed as also including the plural form and vice versa, unless the context indicates otherwise. Thus, it should be noted that as used herein, the singular forms "a," "an," and "the" include plural references unless the context clearly dictates otherwise.

Throughout the description and claims, the terms "comprise", "including", "having", and "contain" and their variations should be understood as meaning "including but not limited to" and are not intended to exclude other components.

Those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure are used. The present invention also covers the exact terms, features, values and ranges etc. in case these terms, features, values and ranges etc. are used in conjunction with terms such as about, around, generally, substantially, essentially, at least etc. (i.e., "about 3" shall also cover exactly 3 or "substantially constant" shall also cover exactly constant).

The term "at least one" should be understood as meaning "one or more", and therefore includes both embodiments that include one or multiple components. Furthermore, dependent claims that refer to independent claims that describe features with "at least one" have the same meaning, both when the feature is referred to as "the" and "the at least one". Features disclosed in the specification, unless stated otherwise, can be replaced by alternative features serving the same, equivalent or similar purpose. Thus, unless stated otherwise, each feature disclosed represents one example of a generic series of equivalent or similar features.

Use of exemplary language, such as "for instance", "such as", "for example" and the like, is merely intended to better illustrate the invention and does not indicate a limitation on the scope of the invention unless so claimed. Any steps described in the specification may be performed in any order or simultaneously, unless the context clearly indicates otherwise.

All of the features and/or steps disclosed in the specification can be combined in any combination, except for combinations where at least some of the features and/or steps are mutually exclusive. In particular, preferred features of the invention are applicable to all aspects of the invention and may be used in any combination. The scope of the disclosure is only limited by the appended patent claims.

## Claims

1. A system for maintaining biological and/or chemical material under controlled environmental conditions, the system comprising:
i. an air-tight enclosure system, within which the biological and/or chemical material is contained and protected from external conditions, the enclosure system enabling observation, access, handling and/or monitoring of the material without exposing the material to external air, the enclosure system having at least one gas inlet and at least one gas outlet;
ii. a gas circulation system, for delivering gas/air or mixture thereof to and from the enclosure system via the at least one gas inlet and the at least one gas outlet;
iii. a motion control system that provides the enclosure system, and any material contained therein, with controlled mechanical pitch-roll orbital wave motion generated by cyclical tilting movement;
iv. a heating system that provides the enclosure system and/or the gas circulation system with controlled heating, thereby controlling temperature within the system.

2. The system of claim 1, further comprising a gas reservoir system, for supplying the gas circulation system with one or more gases in a controlled manner, wherein the gas reservoir system optionally comprises at least one air containment vessel for providing a gas mixture of humidified air.

3. The system of the preceding claim 2, wherein the gas reservoir system further contains one or more of: sensors for determining concentration of one or more gases; ports and valves for injection of pressurized gas; inlet and outlet ports for delivering gas mixtures to and from the gas reservoir system; and one or more heated humidifier.

4. The system of any one of the preceding claims, wherein the enclosure system comprises:
i. an enclosure bottom that is adapted to receive standardized laboratory incubation vessels;
ii. an enclosure lid that is adapted to encircle and meet an upper surface of the standardized laboratory incubation vessel so as to provide an air-tight seal between the enclosure lid and the incubation vessel.

5. The system of the preceding claim 4, wherein the enclosure bottom and/or the enclosure lid are transparent to visible light, so that material within the enclosure can be observed during operation of the system.

6. The system of any one of the preceding two claims 4-5, wherein the enclosure lid comprises at least one air-tight sensor port for enabling the introduction of one or more sensors to measure one or more parameter within the enclosure selected from gas concentration, temperature, pH, pressure and osmolarity.

7. The system of any of the preceding three claims 4-6, wherein the enclosure lid further comprises one or more self-sealing ports, for enabling the insertion or extraction of material into or from the incubation vessel without exposing the interior of the enclosure to external air.

8. The system of any one of the preceding claims 1-7, wherein the circulation system comprises one or more pumps, for moving air or gas mixture from the gas reservoir system to the enclosure system.

9. The system of any one of the previous claims, wherein the heating system comprises at least one of:
i. at least one heating plate that is adapted to meet an enclosure bottom, thereby providing laboratory incubation vessels within the enclosure with controlled heating;
ii. heating means that is integrated into the enclosure lid, for providing the enclosure with controlled heating and preventing condensation on the inner surface of the enclosure lid;
iii. heating means that is integrated into the air containment vessel of the reservoir system;
iv. heating means that is integrated into circulation filters and/or fittings for the circulation filters; and
v. heating means that is integrated into tubing or air/gas passageways in the circulation system.

10. An apparatus for providing mixing of liquid used with chemical reactants or biological culture within an enclosure through a pitch-roll orbital wave motion generated by controlled cyclical tilting movement, the apparatus comprising:
i. at least one foundation having a lower surface and an upper surface for receiving one or more enclosure for holding liquid for chemical reactants and/or biological materials;
ii. a motorized component that is connected to the lower surface of the foundation, the motorized component having means to:
i. tilt the foundation with respect to a horizontal plane; and
ii. drive the foundation in a pitch-roll fashion with respect to the plane of the foundation, while preventing a yaw motion of the foundation in the plane of the foundation,
whereby the motorized component when in use drives the foundation along a pitch-roll orbital path so that any enclosure positioned on the upper surface of the foundation follows the same motion.

11. An apparatus for providing mixing of liquid chemical reactants or biological culture within an enclosure through a pitch-roll orbital wave motion generated by controlled cyclical tilting movement, the apparatus comprising:
i. a base component, comprising a central shaft oriented in a z-direction perpendicular to a flat resting surface and a drive train for providing rotational movement around the central shaft;
ii. a rotational component, the rotational component being operatively connected to the drive train so that the rotational component can rotate around the z-axis, the rotational component having an angled upper portion so as to form an angled wedge when viewed from the side, the upper portion following the rotational movement around the central shaft as the rotational component rotates;
iii. a foundation, the foundation having a lower surface that meets the angled upper portion of the cylindrical rotational component and an upper surface, for receiving one or more enclosure comprising chemical reactants and/or biological liquids;
iv. an arrangement of bearings connecting the foundation to the rotational component so that the bearings form an interface between the rotational component and the lower surface of the foundation; and
v. a linker component that is operatively connected to the platform, the linker component allowing pitch-roll motion of the foundation while preventing yaw motion of the foundation;
wherein when the apparatus is in operation the foundation follows the repositioning of the rotational component without itself rotating around the central shaft, thereby resulting in a pitch roll orbital wave motion of the foundation and any enclosure placed on the foundation.

12. The apparatus of the previous claim, wherein the bearings are arranged on the cylindrical rotational component at or near the upper end thereof, the bearings being aligned with the angled wedge of the upper end.

13. The apparatus of any one of the previous claims 11-12, wherein the linker component comprises an elastic disk with an aperture for connecting to the central shaft, thereby forming a connection between the central shaft and the foundation, the linker providing flexibility to movement of the foundation in terms of pitch and roll, while preventing yaw movement.

14. A method of controlled mixing of liquid chemical reactants or biological culture, the method comprising:
- providing an air-tight enclosure, wherein the enclosure is adapted to be disposed on a solid foundation that is connected to a mechanical assembly for controlled movement of the foundation, and wherein the air-tight enclosure has one or more gas connections for receiving one or more gas mixture without exposing the interior of the enclosure to external air;
- disposing liquid chemical and/or biological material into the air-tight enclosure;
- controlling the composition of gas within the enclosure by supplying gas through the one or more gas connection;
- subjecting the enclosure to a pitch-roll movement without rotating the enclosure, thereby facilitating mixing of chemical and/or biological components within the enclosure while minimizing physical force needed to facilitate mixing.

15. The method of claim 14, wherein the method is carried out using a system according to any one of claims 1-9.
